# FASCICULE DE BREVET EUROPEEN

(11) **EP 1 319 421 B1**
(45) Date de publication et mention de la délivrance du brevet: **16.03.2005**
(21) Numéro de dépôt: 02293086.1
(22) Date de dépôt: 13.12.2002
(51) Int. Cl.: A61N 1/36, A61M 16/00, A61B 5/08

(54) **Dispositif de diagnostic du syndrome d'apnée du sommeil**
Vorrichtung zur Diagnose von Schlafapnoe
Device for diagnosing sleep apnoea syndrome

(30) Priorité: 14.12.2001 FR 0116167
(43) Date de publication de la demande: 18.06.2003
(73) Titulaire: ELA MEDICAL, 92541 Montrouge (FR)
(72) Inventeur: Poezevera, Yann, 91080 Courcouronne (FR)
(74) Mandataire: Dupuis-Latour, Dominique

(56) Documents cités:
- EP-A- 0 970 713
- WO-A-99/65393
- US-A- 5 483 969
- US-A- 6 132 384
- US-A- 6 138 675

## Description

L'invention concerne le diagnostic des troubles respiratoires, plus particulièrement le diagnostic du syndrome d'apnée du sommeil.

Le syndrome d'apnée du sommeil (SAS), plus précisément le syndrome d'apnée obstructive (et non centrale) du sommeil (SAOS) est une affection ayant généralement pour origine une obstruction des voies respiratoires, et qui est susceptible d'entraîner un certain nombre de troubles tels que respiration pénible et insuffisante, troubles du rythme cardiaque, hypertension.

Divers traitements ont été proposés par chirurgie, par voie médicamenteuse, ou encore par maintien d'une pression positive dans les voies respiratoires au moyen d'un masque facial porté pendant le sommeil.

Il a été également proposé de traiter le SAS par stimulation électrique neuro-musculaire des muscles contrôlant les voies aériennes du patient, comme décrit dans le US-A-5 485 851 (Medtronic), ou, plus récemment, par une stimulation particulière du myocarde en cas de SAS détecté, comme décrit par exemple dans les US-A-6 126 611 (Medtronic) ou EP-A-0 970 713 (Ela Médical). Le EP-A-0 970 713 présente l'avantage d'opérer une discrimination entre phases d'éveil et de sommeil, afin de n'appliquer une thérapie que pendant les phases de sommeil et inhiber tout traitement si l'apnée survient pendant une phase d'éveil, car dans ce cas elle n'est normalement pas pathologique.

La présente invention vise un perfectionnement au dispositif décrit dans ce document EP-A-0 970 713.

Plus précisément, ce dispositif connu est un dispositif médical actif comprenant des moyens de mesure de l'activité respiratoire du patient, des moyens de détermination d'un état d'activité, cet état étant susceptible de prendre, en fonction de critères prédéterminés, une valeur représentative d'un état de sommeil du patient, et des moyens d'analyse du signal délivré par les moyens de mesure, aptes à détecter, lorsque ledit état est un état de sommeil, la présence de pauses respiratoires et à produire un signal indicateur d'apnée du sommeil en cas de survenue d'une pause respiratoire de durée supérieure à une première durée prédéterminée.

Le point de départ de la présente invention est la constatation du fait, révélé lors d'études cliniques, qu'en pratique un système mettant en oeuvre un capteur d'activité respiratoire basé sur une variation du volume pulmonaire enregistrée au niveau thoracique peut, dans certaines circonstances, être leurré au cours du sommeil pour des raisons :
- physiologiques internes, par exemple une position corporelle induisant une modification des volumes et/ou de la position des organes concernés,
- cliniques, par exemple du fait d'une respiration d'origine exclusivement abdominale, ou
- externes, par exemple du fait de perturbations électromagnétiques passagères.

Dans ces situations particulières, le dispositif détecte - à tort - la survenue d'une apnée, alors qu'en réalité la respiration est normale mais n'a pas été diagnostiquée comme telle par le dispositif.

L'invention a pour but d'éliminer ces risques de détection de faux positifs dans les dispositifs médicaux pourvus de moyens de détection d'apnées, notamment d'apnées du sommeil.

À cet effet, dans un dispositif du type général connu d'après le EP-A-0 970 713 précité, de façon caractéristique de l'invention les moyens d'analyse comprennent des moyens aptes à inhiber la production dudit signal indicateur lorsque la durée de ladite pause respiratoire est supérieure à une seconde durée prédéterminée, de préférence d'au moins une minute.

Le dispositif peut notamment être un dispositif implantable du type stimulateur cardiaque, défibrillateur, cardioverteur et/ou dispositif multisite, comprenant des moyens de traitement du syndrome d'apnée du sommeil par électrostimulation cardiaque ; les moyens de traitement sont alors inhibés lorsque la durée de la pause respiratoire est supérieure à ladite seconde durée prédéterminée.

Il peut s'agir également d'un dispositif externe alimenté sur le secteur ou sur piles, du type dispositif médical actif.

On va maintenant décrire un exemple de mise en oeuvre de l'invention.

L'activité respiratoire du patient est analysée d'après le signal délivré par un capteur de ventilation-minute, qui est un paramètre à prépondérance physiologique obtenu par une mesure intrathoracique d'impédance.

La mesure de la ventilation-minute est en elle-même bien connue ; elle est opérée entre deux électrodes disposées dans la cage thoracique, ou entre une électrode (par exemple une électrode de stimulation, si le dispositif implanté est un stimulateur cardiaque) et un boîtier du dispositif médical implanté. L'impédance est mesurée par injection d'un courant constant de quelques centaines de microampères, à une fréquence de quelques Hertz, typiquement 8 Hz. Cette technique est par exemple décrite par Bonnet JL et coll., Measurement of Minute-Ventilation with Different DDDR Pacemaker Electrode Configurations, *PACE,* Vol. 21, 98, Part 1, et elle est mise en oeuvre dans les appareils *Chorus RM 7034* d'ELA Médical.

On peut déterminer à partir de ce signal une période respiratoire définie comme étant le laps de temps séparant deux pics d'impédance Les pics correspondent à de fortes impédances obtenues lors de l'inspiration (poumons remplis d'air), et la décroissance de l'impédance correspond à une phase expiratoire.

Les patients souffrant d'apnée du sommeil ont des phases expiratoires normales, car la pression pulmonaire est suffisante pour vaincre l'obstruction, mais leurs phases inspiratoires sont anormales car les poumons ne peuvent pas se remplir d'air. On observe alors un allongement important de la période respiratoire.

De manière connue, on diagnostique une apnée du sommeil lorsque les deux critères suivants sont cumulativement remplis :
- apparition d'une pause respiratoire de durée supérieure à 10 secondes, phénomène qu'il est aisé de détecter par suivi du signal de ventilation-minute, et
- survenue de cette pause pendant une phase de sommeil du patient, car une apnée en état d'éveil ne peut en aucun cas être à l'origine d'un SAS.

La période de sommeil est détectée bien entendu de façon automatique, soit à partir du signal délivré par le capteur de ventilation-minute, soit par un capteur distinct, par exemple un capteur d'activité mesurant un paramètre à prépondérance physique tel qu'un capteur d'accélération interne au boîtier, soit encore par une combinaison des signaux délivrés par ces deux types de capteur.

De façon caractéristique de l'invention si, lors de la détection d'une série d'apnées, une de ces apnées présente une durée anormalement longue, typiquement une durée supérieure ou égale à 1 minute, alors on considère qu'il s'agit d'une fausse apnée.

Le dispositif ne tiendra alors compte de cette apnée :
- ni dans sa détection du syndrome de l'apnée : l'algorithme ne produira pas de signal indicateur de la survenue d'une apnée,
- ni, le cas échéant, dans l'application d'une thérapie : l'algorithme inhibera tout traitement si une telle "fausse apnée" est détectée.

En éliminant cette fausse apnée, on évite en outre de la comptabiliser pour le calcul de l'indice d'apnée (c'est-à-dire le nombre d'apnées par heure de sommeil).

## Revendications

1. Un dispositif médical actif, comprenant :
- des moyens de mesure de l'activité respiratoire du patient,
- des moyens de détermination d'un état d'activité, cet état étant susceptible de prendre, en fonction de critères prédéterminés, une valeur représentative d'un état de sommeil du patient, et
- des moyens d'analyse du signal délivré par les moyens de mesure, aptes à détecter, lorsque ledit état est un état de sommeil, la présence de pauses respiratoires et à produire un signal indicateur d'apnée du sommeil en cas de survenue d'une pause respiratoire de durée supérieure à une première durée prédéterminée,
dispositif **caractérisé en ce que** lesdits moyens d'analyse comprennent des moyens aptes à inhiber la production dudit signal indicateur lorsque la durée de ladite pause respiratoire est supérieure à une seconde durée prédéterminée.

2. Le dispositif de la revendication 1, dans lequel ladite seconde durée prédéterminée est d'au moins une minute.

3. Le dispositif de la revendication 1, dans lequel ledit dispositif est un dispositif implantable actif du type stimulateur cardiaque, défibrillateur, cardioverteur et/ou dispositif multisite, comprenant des moyens de traitement du syndrome d'apnée du sommeil par électrostimulation cardiaque, et dans lequel ces moyens de traitement sont inhibés lorsque la durée de la pause respiratoire est supérieure à ladite seconde durée prédéterminée.

## Claims

1. An active medical device, comprising:
- means for measuring the respiratory activity of the patient;
- means for determining an activity state, which state may take, depending on predetermined criteria, a value representative of a patient sleep state, and
- means for analyzing the signal issued by the detecting means, adapted to detect, when said state is a sleep state, the presence of respiratory pauses and to issue a sleep apnea indicating signal in case of a respiratory pause arising which has a duration longer than a first predetermined duration,
said device being **characterised in that** said analyzing means comprises means adapted to inhibit the issuance of said indicating signal when the duration of said respiratory pause is longer than a second predetermined duration.

2. The device of claim 1, wherein said second predetermined duration is at least one minute.

3. The device of claim 1, wherein said device is an active implantable device of the cardiac pacemaker, defibrillator, cardioverter, and/or multisite device type, including means for treating the sleep apnea syndrome by cardiac electrostimulation, and wherein said treating means are inhibited when the duration of the respiratory pause is longer than said second predetermined duration.

## Patentansprüche

1. Aktive medizinische Vorrichtung, umfassend:
- Mittel zur Messung der Atmungsaktivität des Patienten,
- Mittel zur Bestimmung eines Aktivitätszustands, wobei dieser Zustand im Stande ist, entsprechend vorbestimmten Kriterien einen Wert anzunehmen, der repräsentativ für einen Schlafzustand des Patienten ist, und
- Mittel zur Analyse des Signals, das von den Messmitteln geliefert wird, die dazu geeignet sind, wenn der Zustand ein Schlafzustand ist, die Anwesenheit von Atmungspausen zu entdecken und ein Signal zur Anzeige von Schlaf-Apnoe zu erzeugen im Falle des Auftretens einer Atempause von einer Dauer, die größer ist als eine erste vorbestimmte Dauer,
wobei die Vorrichtung **dadurch gekennzeichnet ist, dass** die Mittel zur Analyse Mittel umfassen, die geeignet sind, die Erzeugung des Signals zur Anzeige zu hemmen, wenn die Dauer der Atempause größer als eine zweite vorbestimmte Dauer ist.

2. Vorrichtung gemäß Anspruch 1, in der die zweite vorbestimmte Dauer mindestens eine Minute ist.

3. Vorrichtung gemäß Anspruch 1, in der die Vorrichtung eine aktive implantierbare Vorrichtung vom Typ Herzschrittmacher, Defibrillator, Kardioverter und/oder eine multizentrische Vorrichtung ist, die Mittel zur Behandlung des Schlaf-Apnoe-Syndroms durch kardiale Elektrostimulation umfasst, und in der diese Mittel zur Behandlung gehemmt sind, wenn die Dauer der Atempause größer ist als die zweite vorbestimmte Dauer.
